# EUROPEAN PATENT APPLICATION

(11) **EP 2 449 907 A2**
(43) Date of publication of application: **09.05.2012**
(21) Application number: 10794330.0
(22) Date of filing: 28.06.2010
(51) Int. Cl.: A44B 11/00, A63B 33/00, A61F 9/02, G02C 5/04

(54) **BUCKLE DEVICE HAVING A RESILIENT BAND, AND ARTICLE HAVING SAME**

(30) Priority: 03.07.2009 KR 20090060677; 23.06.2010 KR 20100059452
(71) Applicant: Lee, Bom Kyu, Yangcheon-gu, Seoul 158-762 (KR)
(72) Inventor: Lee, Bom Kyu, Yangcheon-gu, Seoul 158-762 (KR)
(74) Representative: Oberwalleney, Stephan
(86) International application number: PCT/KR2010/004190
(87) International publication number: WO 2011/002195

(57) **Abstract**

Disclosed is a buckle device that releasably locks an elastic band including a plurality of engaging portions in a lengthwise direction. The buckle device includes a buckle holder and a pair of slide buckles slidably fitted to the buckle holder. The buckle holder includes a pair of locking pins apart from each other and a pair of guide surfaces. A pair of elastic bands are hung on the locking pins respectively. The guide surfaces are inclined in opposite directions with respect to a plane passing through the pair of the locking pins. The slide buckle has a ratchet protrusion configured to engage the engaging portion of the elastic band and a slide surface configured to come into surface contact with the guide surface of the buckle holder. The slide buckle is movable between an unlocked position where the engaging portion pushes the ratchet protrusion in a first direction to allow a movement of the elastic band between the ratchet protrusion and the locking pin, and a locked position where the engaging portion pushes the ratchet protrusion in a second direction opposite to the first direction so that the ratchet protrusion presses the elastic band against the locking pin.

## Description

### Field of the Invention

The present invention relates to a buckle device capable of releasably locking an elastic band. Further, the present invention relates to articles including such a buckle device.

### Background of the Invention

There is known in the art a buckle device capable of releasably locking an elastic band, which has a plurality of protrusions or ridges arranged in a lengthwise direction on its surface. By way of example, such an elastic band may be used as a head strap of a swimming goggle. In such an example, the buckle device is attached to the swimming goggle. The buckle device locks the head strap of an elastic band to secure the swimming goggle to the wearer's head. Further, the buckle device releases the head strap to allow the wearer to take off the swimming goggle.

Korean Patent Application Publication No. 2000-0026927 discloses an example of a buckle device that is used for a swimming goggle and releasably locks an elastic band. In the conventional buckle device disclosed in the aforementioned reference, an elastic band is wound around a locking pin and a slide buckle for locking the elastic band has a protrusion engaging a ridge of the elastic band. When the elastic band is pulled or contracted, the ridges push the protrusion of the slide buckle. The slide buckle releasably locks the elastic band to the locking pin by means of such interaction between the ridge and the protrusion.

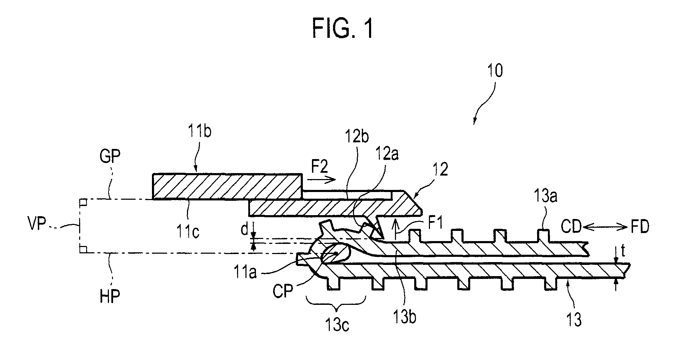

FIG. 1 shows a buckle device of the prior art. A prior art buckle device 10 shown in FIG. 1 releasably locks an elastic band 13 having a plurality of protrusions or ridges 13a on its surface. The buckle device 10 has a slide buckle 12 and a buckle holder (not shown). The buckle holder slidably supports the slide buckle 12. A locking pin 11a, on which the elastic band 13 is hung, is provided in the buckle holder.

The slide buckle 12 has a ratchet protrusion 12a, which the ridge 13a of the elastic band 13 engages, on its bottom surface. The buckle holder has a guide portion 11b guiding the slide buckle 12. The guide portion 11b has a guide surface 11c that contacts a flat surface 12b of the slide buckle 12. The guide surface 11b is situated with no inclination with respect to the locking pin 11a. That is, the guide surface 11c is situated in a guide plane GP intersecting a second plane VP, which is orthogonal to a first plane HP passing through the locking pin 11a, parallel to the first plane HP. Thus, the slide buckle 12 is slidably moved by the ridge 13 a of the elastic band 13 without any change in inclination with respect to the locking pin 11a while being guided by the guide portion 11b and the guide surface 11c.

If the elastic band 13 is pulled in a direction FD wherein an article with the buckle device 10 applied thereto is fastened, then the ridge 13a pushes the ratchet protrusion 12a in the fastening direction FD to thereby move the slide buckle 12 to an unlocked position. In the unlocked position, the ratchet protrusion 12a and the locking pin 11a are apart from each other, and thus, the elastic band 12 passes through between the ratchet protrusion and the locking pin. If the pulled elastic band 13 is set free, then a contractile force of the elastic band 13 moves or contracts the elastic band 13 in a contractile direction CD. Along with the contraction of the elastic band 13, the ridge 13a pushes the ratchet protrusion 12a in the contractile direction CD to thereby move the slide buckle 12 to a locked position. Through such movement, the ratchet protrusion 12a brings a locked portion 13b of the elastic band 13 into contact with the locking pin 11a.

In the locked position of the slide buckle 12, a distance d between a tip end of the ratchet protrusion 12a and the locking pin 11a is less than a thickness *t* of the elastic band 13. Thus, a portion 13c of the elastic band 13, which is located around the locking pin 11a just before the slide buckle 12 goes into the locked position, is wound on the locking pin 11a in a ring shape. Such a ring-shaped portion 13c resists the movement of the slide buckle 12 to the locked position. While the ridge 13a pushes the ratchet protrusion 12a in the contractile direction CD due to the contractile force of the elastic band 13, the ratchet protrusion 12a abuts the ring-shaped portion 13c. Accordingly, to achieve a complete lock of the elastic band 13, the ratchet protrusion must be positioned above the locking pin 11a while compressing an entire upper part of the ring-shaped portion 13c against the locking pin 11a.

Further, in this case, a reaction force F1 strongly acts on the ratchet protrusion 12a, which pushes the ring-shaped portion 13c in the contractile direction CD, from the elastic band 13 toward the ratchet protrusion 12a due to the elasticity of the elastic band 13. The reaction force F1 produces a strong frictional force F2 in a direction opposite to the contractile direction CD between the guide surface 11c and the flat surface 12b of the slide buckle 12. Thus, the slide buckle 12 fails to smoothly move to the locked position.

Further, if the contractile force of the elastic band 13 is less than a force of the ratchet protrusion 12c compressing the ring-shaped portion 13c, then the ratchet protrusion 12a cannot be positioned above the locking pin 11a while compressing the ring-shaped portion 13c. As a result, the lock of the elastic band 13 can be terminated as the ratchet protrusion 12a merely pushes the ring-shaped portion 13c toward the locking pin 11a. This cannot achieve a firm and reliable lock for the elastic band 13. Furthermore, in such a case, the ridge 13a pushing the ratchet protrusion 12a can slip from the ratchet protrusion 12a, and thus, the elastic band 13 cannot achieve as firm fastening as the user wants. Moreover, once the aforementioned phenomenon occurs, the user or wearer must pull and then release the elastic band 13 several times. In some cases, the user must pull a part of the elastic band 13 located below the locking pin 11a. In case a part of the elastic band 130 located below the locking pin 11a is pulled, the ratchet protrusion 12a is pushed by the ridge 13a in the contractile direction CD. As such, the slide buckle 12 can be separated from the locking pin 11a. Then, the locked portion 13b can escape from the locked state.

### Summary of the Invention

The present invention has been devised to solve the aforementioned problems. It is an object of the present invention to provide a buckle device that adjusts an elastic band at a time and reliably locks the elastic band.

To achieve the above and other objects, a buckle device according to an embodiment of the present invention includes: a pair of elastic bands including a plurality of engaging portions in a lengthwise direction; a buckle holder including: a pair of locking pins apart from each other, the pair of the elastic band being hung on the locking pins respectively; and a pair of guide surfaces each inclined in opposite directions with respect to a plane passing through the pair of the locking pins; and a pair of slide buckles each slidably fitted to the buckle holder near the locking pin, the slide buckle including: a ratchet protrusion configured to engage the engaging portion of the elastic band; and a slide surface configured to come into surface contact with the guide surface of the buckle holder. The slide buckle is movable between an unlocked position where the engaging portion pushes the ratchet protrusion in a first direction to allow a movement of the elastic band between the ratchet protrusion and the locking pin, and a locked position where the engaging portion pushes the ratchet protrusion in a second direction opposite to the first direction so that the ratchet protrusion presses the elastic band against the locking pin.

The buckle holder may include a pair of wall portions. The locking pin and the guide surface may be located between the wall portions, and the slide buckle may be slidably fitted between the guide surface and the wall portions.

Further, the buckle holder may include a pair of guide tongues extending toward the locking pin respectively and the slide buckle may include a recess on which the guide tongue is placed. A surface of the guide tongue facing toward the locking pin comprises the guide surface and a surface of the recess comprises the slide surface. Further, an edge of the wall portion facing toward the guide tongue may be inclined at the same inclination as an inclination of the guide surface.

The guide surface is inclined with respect to the plane such that the elastic band is compressed against the locking pin in the locked position of the slide buckle and does not move in the second direction.

Further, the buckle holder may include a push button movable perpendicularly to the plane between the locking pins. The push button may include a pair of inclined surfaces each inclined as opposed to the guide surface and the slide buckle may include an inclined surface contacting the inclined surface of the push button in the locked position. When the push button is moved to the guide surface, the inclined surface of the push button pushes the inclined surface of the slide buckle in the first direction, and thus, the slide buckle may be moved to the unlocked position.

The above-described buckle device may be applied to a swimming goggle having a pair of eyecups with a lens portion. In such a case, the elastic band of the buckle device is joined to an outer end of the eyecup. Further, the buckle device may be applied to a shoe and a swim fin, which are fastened by means of a string. In this case, the elastic bands of the buckle device are coupled to both ends of the string.

A buckle device according to another embodiment of the present invention includes: an elastic band including a plurality of engaging portions in a lengthwise direction; a slide buckle including: a ratchet protrusion configured to engage the engaging portion of the elastic band; and a slide surface; and a buckle holder slidably supporting the slide buckle, the buckle holder including: a locking pin on which the elastic band is hung; and a guide surface configured to come into surface contact with the slide surface of the slide buckle. The guide surface is situated in a guide plane intersecting a second plane, which is orthogonal to a first plane passing through a center of the locking pin, obliquely with respect to the first plane. The slide buckle is movable between an unlocked position where the engaging portion pushes the ratchet protrusion in a first direction to allow a movement of the elastic band between the ratchet protrusion and the locking pin, and a locked position where the engaging portion pushes the ratchet protrusion in a second direction opposite to the first direction so that the ratchet protrusion presses the elastic band against the locking pin.

The buckle holder may include a guide portion extending toward the locking pin and the slide buckle may include a recess on which the guide portion is placed. A surface of the guide portion facing toward the locking pin comprises the guide surface and a surface of the recess comprises the slide surface.

The above-described buckle device may be applied to a swimming goggle including a pair of eyecups with a lens portion respectively. The buckle device may be attached to an outer end of each eyecup. The elastic bands of the buckle devices are integrally formed.

In the above-described buckle devices, the engaging portion of the elastic band may include one selected from a protrusion, a ridge and a groove, which extend in a width direction of the elastic band.

According to the buckle device of the invention, when the slide buckle is moved from the unlocked position to the locked position, the ratchet protrusion slantingly or obliquely compresses a locked portion of the elastic band against the locking pin. Thus, the slide buckle can slidably move from the unlocked position to the locked position with low frictional force and without any restraint. Further, when the slide buckle goes into the locked position, the slippage of the elastic band on the slide buckle does not occur and the elastic band can be firmly and reliably fixed to the locking pin.

### Brief Description of the Drawings

FIG. 1 is a sectional view showing a prior art buckle device.
FIG. 2 is a sectional view showing a buckle device according to a first embodiment with a slide buckle in an unlocked position.
FIG. 3 is a sectional view showing the buckle device according to the first embodiment with the slide buckle in a locked position.

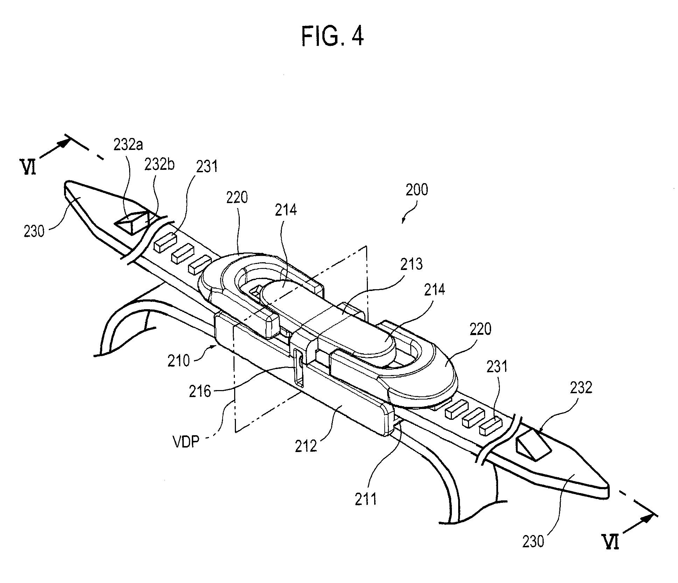

FIG. 4 is a perspective view showing a buckle device according to a second embodiment.
FIG. 5 is an exploded perspective view of the buckle device shown in FIG. 4.
FIG 6 is a sectional view taken along the line VI-VI in FIG. 4.
FIG. 7 is a front view showing a buckle holder.
FIG. 8 is a front view showing a slide buckle.
FIG 9 is a bottom view of the slide buckle.
FIG. 10 is a sectional view similar to FIG 6 wherein the slide buckle is in an unlocked position.
FIG. 11 is a sectional view similar to FIG 6 wherein the slide buckle is in a locked position.
FIG. 12 is a front view showing another embodiment of the slide buckle.
FIG. 13 is a front view showing another embodiment of the buckle holder.
FIG. 14 is a sectional view similar to FIG. 6 showing another embodiment of an elastic band.
FIG. 15 is a perspective view of a swimming goggle to which the buckle device shown in FIG. 4 is applied.
FIG. 16 is a perspective view of a shoe to which the buckle device shown in FIG. 4 is applied.
FIG. 17 is a perspective view of a swim fin to which the buckle device shown in FIG. 4 is applied.
FIG. 18 is a plan view of a swimming goggle to which the buckle device shown in FIGS. 2 and 3 is applied.
FIG. 19 is a sectional view showing one of eyecups and a buckle device in the swimming goggle shown in FIG. 18.

### Details of the Invention

Embodiments of a buckle device and embodiments of articles to which such a buckle device is applied are described with reference to the accompanying drawings. Like reference numerals in the drawings denote like elements or parts.

In the descriptions provided below, the directional term "outward" or "outer" refers to a direction wherein an elastic band is moved from a buckle device to fasten or tie an article with the buckle device applied thereto, while the directional term "inward" or "inner" refers to a direction opposite to the outward or outer direction.

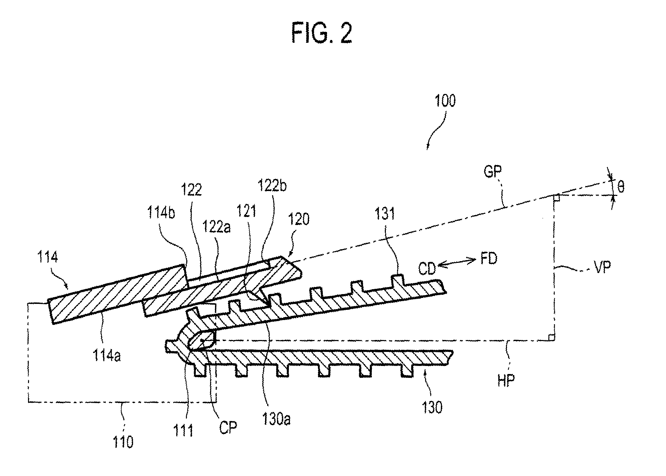

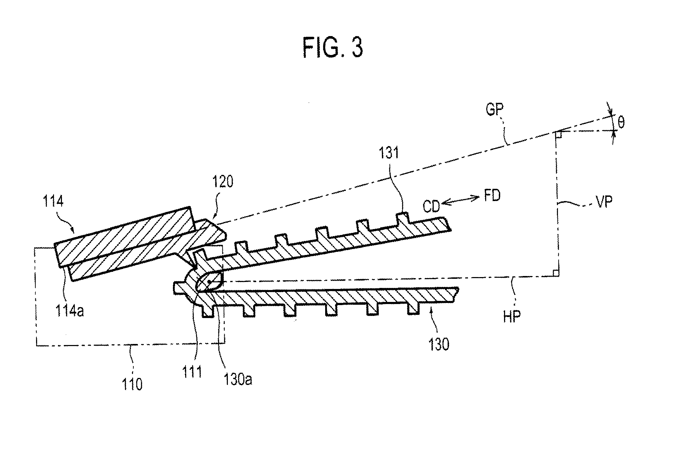

FIGS. 2 and 3 show a buckle device according to a first embodiment. The buckle device 100 according to the first embodiment is configured to releasably lock a piece of an elastic band 130. The elastic band 130 has, on its surface, a plurality of protrusions or ridges 131 that serve as an engaging portion when it is being locked to the buckle device 100. The buckle device 100 includes a buckle holder 110 and a slide buckle 120. The buckle holder constitutes a body of the buckle device and has a locking pin 111 on which the elastic band 130 is hung. The slide buckle is slidably fitted to and held in the buckle holder 110.

The buckle holder 110 slidably supports the slide buckle 120. The buckle holder 110 has a guide portion 114 for guiding a slide movement of the slide buckle 120. The guide portion 114 extends toward the locking pin 111. The guide portion 114 has a guide surface 114a, on which the slide buckle 120 is guided, on a surface facing toward the locking pin 111. The slide buckle 120 is slidably fitted to the buckle holder 110 in contact with the guide portion 114.

The slide buckle 120 has, on its bottom surface, a ratchet protrusion 121 configured to contact the ridge 131 of the elastic band 130. The ratchet protrusion 121 has a shape of a barb or wedge, a tip end of which leans toward a fastening direction FD (a direction in which the elastic band 130 is pulled or stretched to fasten or tie the article with the buckle device 100 applied thereto). Further, the slide buckle 120 has a recess 122 on which the guide portion 114 is placed. A surface of the recess 122 is a slide surface 122a that comes into surface contact with the guide surface 114a. While guided on the guide surface 114a, the slide buckle 120 is slidably moved between an unlocked position where the ratchet protrusion 121 of the slide buckle 120 is moved away from the locking pin 111 to thereby allow the movement of the elastic band 130 between the ratchet protrusion 121 and the locking pin 111 (*see* FIG. 2), and a locked position where the ratchet protrusion 121 of the slide buckle 120 is situated above the locking pin 111 to thereby press the elastic band 130 against the locking pin 111 so that the elastic band 130 is fixed to the locking pin 111 as compressed to some extent (*see* FIG. 3).

One end portion of the elastic band 130 may be joined to an article that can be fastened or tied by means of the elastic band 130. Further, a middle part of the elastic band 130 may pass through the buckle holder 110 and a terminal end portion of the elastic band 130 may be a free end. The elastic band 130 extends through the buckle holder 110 of the buckle device 100. Specifically, the elastic band 130 extends through an internal space of the buckle holder 110 as wound on the locking pin 111 provided in the buckle holder 110. The elastic band 130 has a shape of an elongated string or band. The elastic band 130 is made from an elastomer material or a plastic material having elasticity or expandability/contractibility.

The elastic band 130 may be pulled or stretched in the fastening direction FD as wound on the locking pin 111. During the movement in the fastening direction FD, the ridge 131 of the elastic band 130 pushes the ratchet protrusion 121 outwardly of the buckle holder 110 (in a first direction) to move the slide buckle 120 to the unlocked position. If the elastic band 130 is pulled or stretched to some extent and then released (e.g., when the user pulls the elastic band 130 from the buckle holder 110 in the fastening direction FD and then sets it free), then the elastic band 130 is moved or contracted due to its elasticity or contractibility in a contractile direction CD (a direction wherein the elastic band 130 is closely contacted to the article with some contractile force) to fix the elastic band 130 to keep the article fastened or tied. During the movement in the contractile direction CD, the ridge 131 of the elastic band 130 pushes the ratchet protrusion 121 inwardly of the buckle holder 110 (in a second direction opposite to the first direction) to move the slide buckle 120 to the locked position.

The slide buckle 120 has a stopper element that restricts the slide buckle from moving further inward than the locking pin 111 beyond the locked position. That is, if the ridge 131 pushes the slide buckle 120 and the ratchet protrusion 121 is moved further inward than the locking pin 111, then lock of the elastic band 130 cannot be achieved. For purposes of avoiding such a situation, the slide buckle 120 has a stopper portion 122b abutting an outer end 114b of the guide portion 114 as said stopper element. The stopper portion 122b is positioned to abut the outer end 114b such that the ratchet protrusion 121 cannot be moved inwardly of the buckle holder 211 beyond the locking pin 111.

Further, the guide surface 114a of the buckle holder 110 is inclined with respect to the locking pin 111. Specifically, the guide surface 114a is situated in a guide plane GP that slantingly intersects a second plane VP, which is orthogonal to a first plane HP passing through a central point CP of the locking pin 111, at a predetermined angle θ with respect to the first plane HP. Said angle θ may have a value ranging from 1 degree to 25 degrees. Preferably, said angle θ has a value ranging from 3 degrees to 10 degrees. Said angle θ may be selected to achieve reliable lock of a locked portion 130a of the elastic band.

With the above-described arrangement, when assuming the first plane HP as a reference, a distance between the ratchet protrusion 121 and the locking pin 111 in the unlocked position shown in FIG. 2 is greater than a distance between the ratchet protrusion 121 and the locking pin 111 in the locked position shown in FIG. 3. Thus, when the slide buckle 120 is slidably moved from the unlocked position shown in FIG. 2 to the locked position shown in FIG. 3, for example, when the elastic band 130 is moved in the contractile direction CD in FIG. 2, the slide buckle 120 approaches the locking pin 111 obliquely with respect to the locking pin 111 (obliquely with respect to the first plane HP).

Due to a greater space in the unlocked position and the oblique movement of the slide buckle 120, the elastic band 130 does not form a portion ringing around the locking pin 111 when the ratchet protrusion 121 approaches the locked position. Further, when going into the locked position, the ratchet protrusion 121 obliquely compresses the portion 130a of the elastic band 130, which is to be locked to the locking pin 111. Thus, no force acts from the elastic band 130 to the slide buckle 120 in a direction perpendicular to the guide surface 114a in the vicinity of the locked position. Thus, the slide buckle 120 can move to the locked position without any restraint. Further, since the ratchet protrusion 121 approaches the locking pin 111 while compressing the elastic band 130 obliquely or slantingly with respect to the locking pin 111, the elastic band 130 can reliably move to the locked position at a time. Further, if a part of the elastic band 130 located below the locking pin 111 is pulled while the locked portion 130a is locked to the locking pin 111, then the ratchet protrusion 121 is pushed in the contractile direction CD by the ridge 131 and the slide buckle 120 can be somewhat moved inwardly of the locking pin 111. In such a case, the slide buckle 120 further compresses the locked portion 130a obliquely with respect to the locking pin 111. Thus, the more the part of the elastic band 130 located below the locking pin 111 is pulled, the more firmly the locked portion 130a is locked.

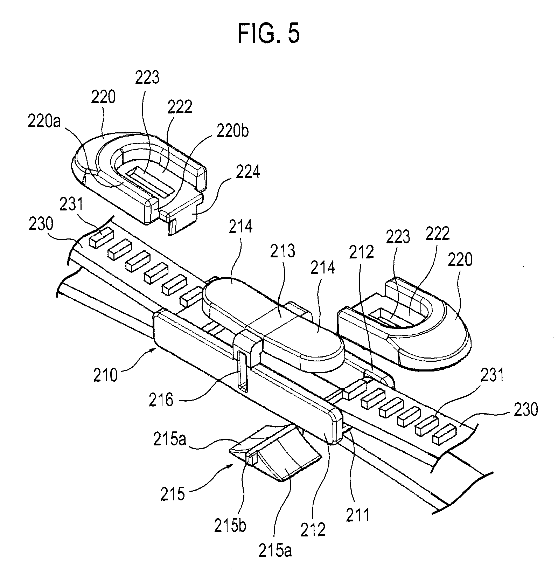

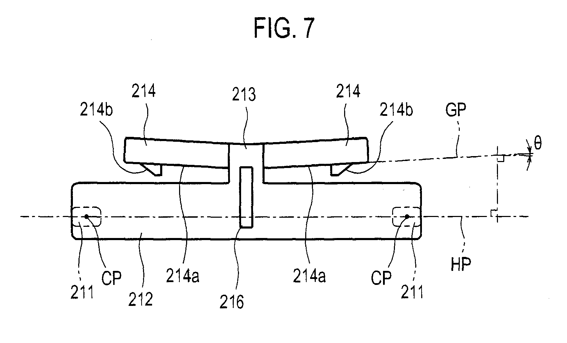

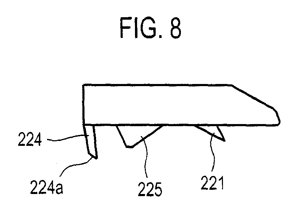

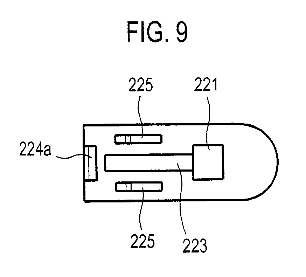

FIGS. 5 to 11 show a buckle device according to a second embodiment of the present invention.

The buckle device 200 according to this embodiment is configured to releasably lock a pair of or two pieces of elastic bands 230 at its both sides respectively. The buckle device 200 includes a buckle holder 210 and a pair of slide buckles 220 that are slidably coupled to the buckle holder 210 and locks each elastic band 230 to the buckle holder 210. The buckle holder 210 includes a pair of locking pins 211 to which the elastic bands 230 are hung and locked. The buckle device 200 is configured to be symmetrical with respect to a vertical plane VDP that passes through the longitudinal middle of the buckle holder 210 and is orthogonal to a first plate HP passing through central points of the locking pins 211. The locking pins 211 are apart from each other with the vertical plane VDP centered therebetween.

The buckle holder 210 constitutes a body of the buckle device 200 and is formed in the shape of a generally rectangular frame. Said frame includes a pair of wall portions 212 having a rectangular plate shape and a bridge portion 213 connecting the wall portions 212 with certain spacing therebetween. The bridge portion 213 somewhat upwardly protrudes from a midway upper edge of the wall portion 212 and connects the wall portions 212 to each other. In another embodiment, the buckle holder 210 may include a connection member that connects lower edges of the wall portions 212 and is integrally formed with the wall portions 212.

The locking pins 211 are located in the vicinity of both longitudinal ends of the wall portions 212 between the wall portions 212. In this embodiment, the locking pins 211 are integrally formed with the wall portions 212. In another embodiment, the locking pins 211 may be provided separately from the buckle holder 210 and be fitted to the wall portions 212. A cross-sectional shape of the locking pin 211 is close to a rectangle with rounded corners. In another embodiment, the cross-sectional shape of the locking pin 211 may include an ellipse, elongated ellipse, circle and the like.

A pair of the elastic bands 230 have a band shape. A cross-sectional shape of the elastic band 230 is generally rectangular throughout its entirety. Alternatively, a cross-sectional shape of a part of the elastic band, which passes through the buckle holder 210, may be rectangular, while a cross-sectional shape of the rest may be circular or elliptical. The elastic band 230 is made from an elastomer material or plastic material having elasticity or expandability/contractibility. The elastic band 230 extends through an internal space defined by the wall portions 212 as hung on the locking pin 212.

The pair of the elastic bands 230 may be disposed between the buckle device 200 and an article in such a manner that both end portions of a piece of a long elastic band extend through both sides of the buckle holder 210 and a middle part thereof is joined to the article with the buckle device 200 applied thereto. Alternatively, the pair of the elastic bands 230 may comprise one end portion of each of two pieces of elastic bands. The pair of the elastic bands may be disposed between the buckle device 200 and the article in such a manner that each end portion may extend through the respective sides of the buckle holder 210 and the opposite end portions are joined to the article.

The elastic band 230 has a plurality of engaging portions, which serve as an engaging element when the elastic band is being locked to the buckle holder 210, within a predetermined range from one end toward the opposite end. In this embodiment, said engaging portions include a plurality of protrusions or ridges 231. The protrusions or ridges 231 extend in a width direction of the elastic band 230. The protrusions or ridges 231 are arranged at approximately equal spacing in a lengthwise direction of the elastic band 230. The elastic band 230 has a stopper 232 near its one end. The stopper 232 has an inclined surface 232a inclined toward the one end and a vertical surface 232b vertical relative to the inclined surface 232a. A height of the stopper 232 is greater than a height of the ridge 231. The stopper 232 allows the elastic band 230 not to easily come out through between the slide buckle 220 and the locking pin 211 after the elastic band is inserted to the buckle holder 210.

The buckle holder 210 includes a guide surface for guiding a movement of the slide buckle 220 into or out of the buckle device 200. In this embodiment, said guide surface includes surfaces of a pair of guide tongues 214 (bottoms surfaces of the guide tongues 214), which face toward the locking pin 211. The guide tongues extend from the bridge portion 213 outwardly in opposite directions. The guide tongue 214 has a shape of a generally U-shaped plate. The guide tongue 214 is inclined an a predetermined angle with respect to the upper edge of the wall portion 212 or the locking pin 211 located at either end of the wall portion 212. Since the guide tongue 214 extends from the bridge portion 213, a certain space is defined between the guide tongue 214 and the upper edge of the wall portion 212. The slide buckle 220 is moved into or out of the buckle holder 210 as fitted to said space.

As described above, the guide tongue 214 has a flat guide surface 214a, on which the slide buckle 220 is guided, at its bottom surface. Each guide surface 214a is inclined with respect to the respective locking pins 211. Specifically, each guide surface 214a is situated in respective guide planes GP1, GP2. The guide planes are inclined in opposite directions at a predetermined angle θ with respect to the first plane HP passing through the central points CP of the pair of locking pins 211. The inclination of the guide surface 214a may be defined in an alternative way. That is, one of the guide surfaces 214a is situated in the guide plane GP1 that slantingly intersects the second plane VP, which is orthogonal to the first plane HP passing through the central points CP of the locking pins 211, at the predetermined angle θ with respect to the first plane HP. Said angle θ is determined such that a locked portion 230a of the elastic band 230 is compressed without the movement of the elastic band 230 in the contractile direction CD when the locked portion 230a is pinched between the locking pin 211 and a tip end of a ratchet protrusion 221. Said angle θ may be selected in the range of degree to 25 degrees (preferably, 3 degrees to 10 degrees). When said angle θ is too great, the ridge 231 cannot precisely contact the slide buckle 220 during the movement to the locked position. When said angle θ is too small, the elastic band 230 may form a portion ringing around the locking pin 211 in the vicinity of the locked position. Further, the guide tongue 214 has a downwardly protruding stopper 214b on the guide surface 214a. The stopper 214b is positioned to be fitted to a slot 223 of the slide buckle 220 (this will be described below) when the slide buckle 220 is assembled to the buckle holder 210.

The slide buckle 220 is held in the buckle holder 210 as fitted to the space between the upper edge of the wall portion 212 and the guide tongue 214. The slide buckle 220 has the ratchet protrusion 221 on its bottom surface. The ratchet protrusion is configured to engage the ridge 231 of the elastic band 230. The ratchet protrusion 221 has a shape of a barb or wedge, the tip end of which leans toward a fastening direction FD (a direction in which the elastic band 230 is pulled or stretched to fasten or tie the article with the buckle device 200 applied thereto). By way of another example, the ratchet protrusion 221 may have a cross-sectional shape of a right-angled triangle with its hypotenuse facing toward the fastening direction FD, or a cross-sectional shape of a simple rectangle.

The elastic band 230 may be pulled or stretched in the fastening direction FD as hung on the locking pin 211. The fastening direction FD is a direction wherein the elastic band 230 is moved to fasten the article when a part of the elastic band 230 located below the locking pin 211 and another part of the elastic band 230 located above the locking pin are situated at an acute angle therebetween. As the elastic band 230 is pulled or stretched in the fastening direction FD, the buckle holder 210 is brought into close contact with the article and the article is fastened due to the stretched elastic band 230. If the elastic band 230 is pulled in the fastening direction FD, the elastic band 230 is moved or stretched in the fastening direction FD and the ridge 231 pushes the ratchet protrusion 221 in the first direction in engagement with the ratchet protrusion 221. As a result, the slide buckle 220 is moved outwardly of the buckle device 200 and thus can be moved to or maintained in the unlocked position. By contrast, if releasing the pull or stretch of the elastic band 230, then the elastic band 230 is contracted due to its elasticity or contractibility in the contractile direction CD (a direction in which the elastic band 230 is closely contacted to the article with some contractile force). During such contraction, one of the ridges engages the ratchet protrusion 221 and pushes the ratchet protrusion 221 in the second direction (a direction opposite to the first direction). Thus, the slide buckle 220 is moved inwardly of the buckle device 200 in concomitance with the contractile force of the elastic band 230. Such inward movement moves the slide buckle 220 to the locked position.

The slide buckle 220 has a slide surface configured to come into surface contact with the guide surface 214a of the buckle holder 210. In this embodiment, said slide surface includes a surface of a U-shaped recess 222 formed on the slide buckle 220. A contour of the recess 222 is similar to a contour of the guide tongue 214. The surface of the recess 222 is substantially flat. Thus, the slide buckle 220 is slidably guided along the guide surface 214a of the guide tongue 214 with the guide tongue 214 fitted to the recess 222.

An elongated slot 223 is formed along the first and second directions in the middle of the recess 222. The stopper 214b of the guide tongue 214 is fitted to the slot 223. The stopper 214b abuts an inner end of the slot 223 to thus prevent the slide buckle 220 from separating from the buckle holder 210. Further, the slide buckle 220 has a stopper element preventing the slide buckle from moving further inward than the locking pin 211 beyond the locked position. In this embodiment, said stopper element includes inner ends 220b of both lateral portions 220a of the slide buckle 220, which defines the recess 222. The inner end 220b is positioned to abut the bridge portion 213 such that the ratchet protrusion 221 does not move inwardly of the buckle holder 211 beyond the locking pin 211.

The slide buckle 220 has a pressing plate 224, which extends downwardly vertically, at its inner end. A tip end of the pressing plate 224 has an inclined surface 224a. The inclined surface 224a interacts with a push button (this will be described below) to move the slide buckle 220 to the unlocked position. The slide buckle 220 has, on its bottom surface, a pair of guide fins 225 with the slot 223 therebetween. A distance between the guide fins 225 is slightly less than a distance between the wall portions 212 of the buckle holder. The guide fins 225 are situated between the wall portions 212 to further prevent lateral shake of the slide buckle 220.

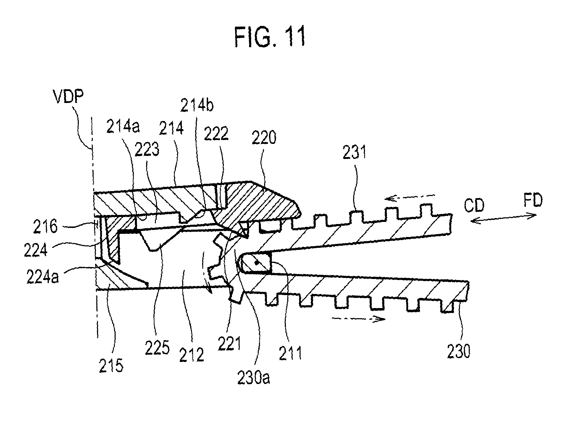

When the slide buckle 220 is in the locked position (*see* FIG. 11), the ratchet protrusion 221 compresses the elastic band 230 against the locking pin 221 and thus the elastic band 230 is locked between the locking pin 211 and the ratchet protrusion 221 as compressed to some extent. In this locked position, an expanding force of the compressed elastic band 230 strongly presses the slide buckle 220 toward the guide tongue 214. To facilitate the movement of the slide buckle 220 to the unlocked position from such a state, the buckle holder 210 includes an element for pushing the slide buckle 220 outwardly of the buckle holder 210. In this embodiment, said element comprises a push button 215 disposed in the buckle holder 210 so as to vertically move along the vertical plane VDP.

The push button 215 includes an inclined surface 21 spa inclined opposite to the guide surface 214a and has a cross-sectional shape of a general equilateral triangle. The inclined surface 215a may include a flat surface or a smoothly curved concave surface. To guide a vertical movement of the push button 215, the push button 215 has insertion protrusions 215b protruding from its upper end toward the wall portion 212 of the buckle holder. Further, the buckle holder 210 has guide slots 216, to and by which the insertion protrusions 215b are fitted and guided. The guide slot 216 extends from the vicinity of the lower end of the wall portion 212 to the vicinity of the upper end of the bridge portion 211 along the vertical plane VDP. Thus, the push button 215 is vertically movable in the buckle holder 210 with its insertion protrusions 215b fitted to the guide slots 216. By way of another example, the buckle holder 210 may include guide grooves that are vertically formed on inner surfaces of the wall portions 212. The insertion protrusions 215b of the push button may be fitted to the guide grooves.

In the locked position, the push button 215 is located near the lower end of the wall portion 212, while the inclined surface 224a of the pressing plate 224 of the slide buckle 220 is located above the inclined surface 215a of the push button 215. If pushing the push button 215 upward (toward the slide buckle 220), then a force toward the outside of the buckle holder 210 acts on the inclined surface 224a of the pressing plate 224 due to reaction caused by the upward movement of the push button 215. Such a force can strongly move the slide buckle 220 toward the unlocked position against the pressing force of the elastic band 230.

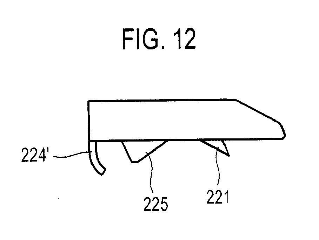

The pressing plate 224 of the slide buckle 220 may have another configuration for purposes of smooth slide contact on the inclined surface 215a of the push button 215. For example, as shown in FIG. 12, the pressing plate 224' may be configured to be convexly curved toward the inclined surface 215a.

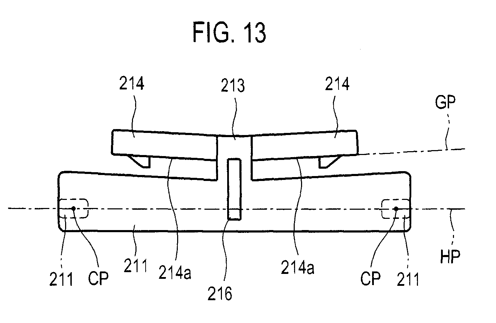

As shown in FIGS. 5 to 7, the guide tongue 214 is inclined with respect to the locking pin 211. Further, the upper edge of the wall portion 212 of the buckle holder 210 is substantially parallel to the first plate HP. The slide buckle 220 is slidably movable into or out of the buckle holder 210 while the lateral portions 220a of the slide buckle 220 are situated in the space between the upper edge of the wall portion 212 and the guide tongue 214. When the elastic band 230 is wound on the locking pin 211, an outer end of the slide buckle 220 is somewhat lifted due to the thickness of the elastic band 230 and the surface of the recess 222 (slide surface) and the guide surface 214a are in close contact with each other. Under such close contact, the slide buckle 220 is moved between the locked position and the unlocked positions while guided by the guide surface 231a. To guide the slide movement of the slide buckle 220, the buckle holder 210 may include an additional guide portion. For example, as shown in FIG. 13, the left and right upper edges of the wall portions 212 of the buckle holder 210 may be slantingly formed at the same angle as the inclination angle θ of the guide surface 214a with respect to the first plane HP. In such a case, the lateral portions 220a of the slide buckle 220 is situated between the guide tongue 214 and the upper edge of the wall portions 212 with little clearance and the slide buckle 220 is slidably movable while guided by both the guide surface 214a and the upper edge of the wall portion 212.

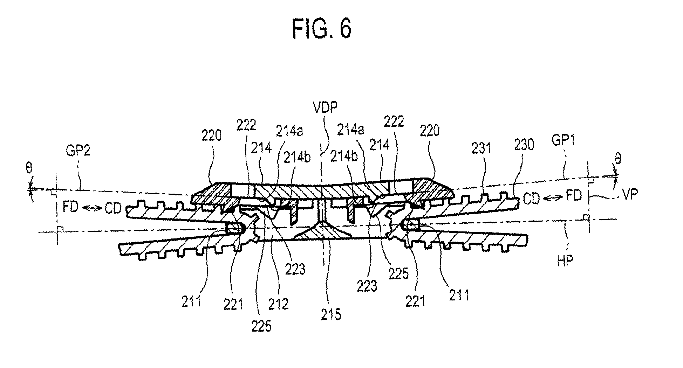

With reference to FIGS. 6, 10 and 11, descriptions are made as to examples of locking and unlocking the elastic band in the buckle device 200 configured as described above.

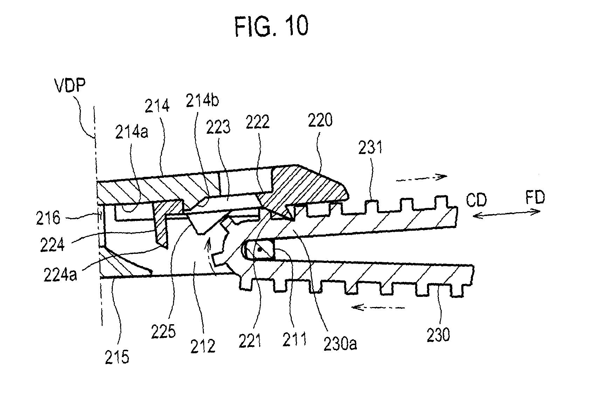

In a state where the elastic band 230 is movable without any restraint or immovable between the ratchet protrusion 221 and the locking pin 211, the user grasps the one end portion of the elastic band 230 and pulls it in the fastening direction FD. Then, the elastic band 230 is moved around the locking pin 211 in the fastening direction FD. Further, if the elastic band 230 is further pulled when the article is fastened or tied to some extent due to the movement of the elastic band 230, then the elastic band 230 is stretched in its lengthwise direction. During such movement and stretch, the ridges 231 of the elastic band push the ratchet protrusion 221 of the slide buckle outwardly (i.e., in the first direction). Thus, push or bump of the ridge 231 moves the slide buckle 220 to the unlocked position shown in FIG. 10 (i.e., the position of the slide buckle 230 wherein the elastic band 230 is movable without any restraint between the locking pin 211 and the ratchet protrusion.221) or maintains the slide buckle in the unlocked position. In this case, the stopper 214b of the guide tongue 214 engages the inner end of the slot 223 of the slide buckle, thereby preventing the slide buckle 230 from separating from the buckle holder 210.

If the stretched elastic band 230 is released (or the user sets the elastic band 230 free) after fastening the article to some extent, then the elastic band 230 is moved in the contractile direction CD due to its elasticity or contractibility and goes around the locking pin 211. During such contractile movement, the ridge 231 of the elastic band 230 bumps into the ratchet protrusion 221 from outside to inside and then pushes the ratchet protrusion 221 toward inside (i.e., in the second direction). Thus, the slide buckle 220 is strongly pushed inwardly of the buckle holder 210 due to such push or bump. If the ratchet protrusion 220 is pushed by the ridge 231 and is located near the locking pin 211, then the locked portion 230a of the elastic band 230, which is between the ridge 231 pushing the ratchet protrusion 221 and another ridge 231 located further inward than said ridge 231, is brought into contact with the locking pin 211. At this time, the contractile force of the elastic band 230 continues to act and the ridge 231 contacting the ratchet protrusion 221 continues to apply a force to the ratchet protrusion toward the inside of the buckle holder 210. In the locked position, the shortest distance between the ratchet protrusion 221 and the locking pin 211 is less than the thickness of the elastic band 230. Further, since the guide surface 214a is inclined with respect to the locking pin 211, the slide buckle 220 and the ratchet protrusion 221 obliquely approach the locking pin 211. Since the ratchet protrusion 221 pushed by the ridge 231 along with the contraction of the elastic band 230 obliquely approaches the locking pin 211, the locked portion 230a of the elastic band is pinched between the locking pin 211 and the ratchet protrusion 221 while compressed to some extent against the locking pin 211 by the obliquely approaching ratchet protrusion 221. Such pinch produces strong friction forces in a direction opposite to the contractile direction of the elastic band 230 between the locked portion 230a and the locking pin 211 and between the locked portion 230a and the ratchet protrusion 221. If said friction forces are greater than the contractile force of the elastic band 230, then the locked portion 230a stops between the locking pin 211 and the ratchet protrusion 221 and the elastic band 230 is fixed or locked to the locking pin 211, as shown by the locked position in FIG 11. Further, in the locked position, the inclined surface 224a of the pressing plate 224 of the slide buckle 220 is located above the inclined surface 215a of the push button 215.

As described above, the guide surface 214a of the buckle holder 210 is inclined at the predetermined angle θ with respect to the first plane HP passing through the pair of the locking pins 211. Further, when assuming the first plane HP as a reference, the distance between the ratchet protrusion 221 and the locking pin 211 in the unlocked position is greater than the distance between the ratchet protrusion 221 and the locking pin 211 in the locked position. In addition, the distance between the ratchet protrusion 221 and the locking pin 211 in the locked position is less than the thickness of the elastic band 230. Further, in the unlocked position, the outer end of the slide buckle 220 is somewhat lifted due to the elastic band 230 and the slide buckle is held in the buckle holder 210 with the recess 222 in contact with the guide surface 214a.

With the above-described arrangement of the locking pin 211, the guide surface 214a and the slide buckle 220, the slide buckle 220 does not move to the locked position in such a manner that it slides parallel to the first plane HP. The slide buckle 220 slides obliquely with respect to the locking pin 211, i.e., obliquely or slantingly with respect to the first plane HP and then enters the locked position. Further, when the slide buckle 220 enters the locked position, the elastic band 230 does not form the portion that is pushed by the ratchet protrusion 221 and thereby rings around the locking pin 211. Thus, when the slide buckle 220 enters the locked position, it does not occur that a force perpendicular to the guide surface 231a is applied from the elastic band 230 to the slide buckle 220 and a great friction force is generated between the slide buckle 220 and the guide surface 214a. Moreover, it does not occur that the slide buckle 220 cannot slide inwardly of the buckle holder 210 due to catch of the slide buckle on a portion of the elastic band 230 wound around the locking pin 211 and the ridge 231 slips from the ratchet protrusion 221 due to such catch. Further, the inclination of the guide surface 214a to the locking pin 211 and the oblique engagement of the ratchet protrusion 221 caused by the inclination allow the slide buckle 220 to smoothly slide to the locked position without any restraint and the ratchet protrusion 221 to rapidly compress the locked portion 230a, thereby firmly fixing the elastic band 230. Further, if a part of the elastic band 230 below the locking pin 211 is pulled after the locked portion 230a is locked to the locking pin 211, then the slide buckle 220 is moved along the guide surface 214a and the ratchet protrusion 221 further compresses the locked portion 230a obliquely with respect to the locking pin 211, thus providing stronger lock to the locked portion 230a.

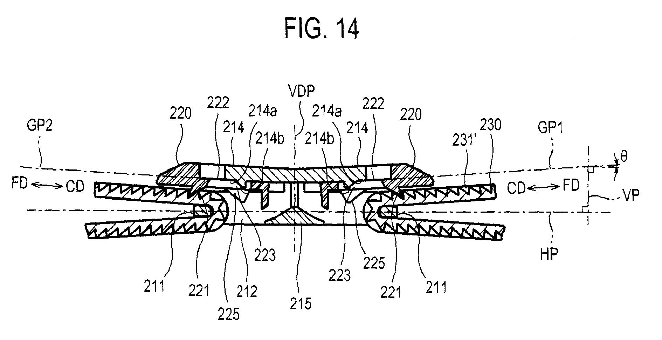

In the foregoing embodiments of the buckle device, it is illustrated that the elastic band 130, 230 has a plurality of protrusions or ridges 131, 231 on its surface. In other embodiment, the elastic band 130, 230 may have a plurality of grooves extending in a width direction on its surface. FIG. 14 shows an example of such an elastic band. As shown in FIG. 14, the elastic band 230' has a plurality of grooves 231' on its surface. The grooves 231' extend in a width direction of the elastic band 230' and are arranged at equal spacing in a lengthwise direction of the elastic band. A cross-sectional shape of the groove 231' may include a triangle, a right-angled triangle, a rectangle, etc.

In the foregoing embodiments of the buckle device, it is illustrated that a planar shape of the ridge 131, 231 of the elastic band 130, 230 is a rectangle. The planar shape of the ridge 131, 231 may include a parallelogram or an elongated ellipse.

In the foregoing embodiments of the buckle device, it is illustrated that the slide buckle 220 is fitted to the space between the wall portions 212 and the guide tongue 214 protruding from the wall portions 212. In other embodiment, the guide tongue 214 may be formed to be flush with the upper edge of the wall portion 212. Further, stepped portions, on which the lateral portions of the slide buckle 220 are placed, may be formed on the inner surfaces of the wall portions 212 and the slide buckle 220 may be placed on said stepped portions.

Further, in the foregoing embodiments of the buckle device, it is illustrated that the pair of the slide buckles 220 are guided by the guide tongues 214 extending from the bridge portions 213 in opposite directions. In other embodiment, guide portions similar to the guide tongue 214 are provided in the buckle holder 210 in such a manner that said guide portions extend from the respective ends of the buckle holder 210 toward the inside of the buckle holder 210.

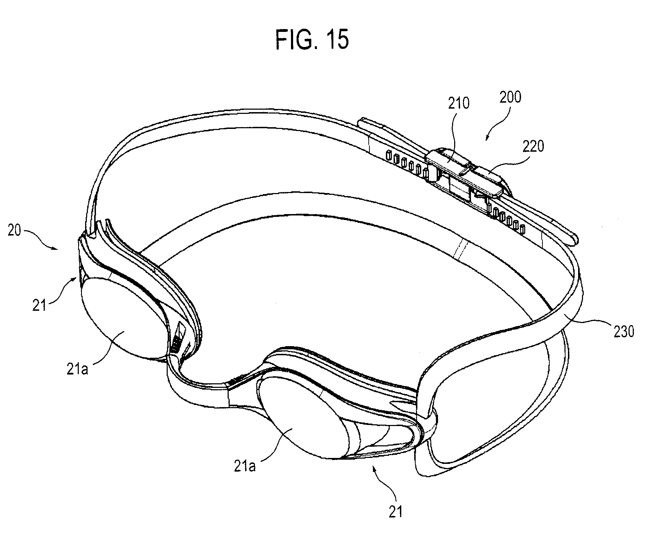

The buckle device 200 according to the foregoing second embodiment has two slide buckles 220 at the respective sides of the buckle holder 210. Both end portions of a piece of the elastic band or each end portion of two pieces of elastic bands extend as hung on the locking pins located in the respective sides of the buckle holder 210. Thus, as to the buckle device 200, the elastic band 230 joined to the article can be adjusted at a time by pulling and releasing two end portions of the elastic band 230. FIGS. 15 to 17 show examples of articles to which such a buckle device 200 is applied.

FIG. 15 shows a swimming goggle as an example of the article to which the buckle device 200 is applied. The swimming goggle 20 includes a pair of eyecups 21 with a lens portion 21a. In the swimming goggle 20, an elastic or expandable/contractible head strap engirding the wearer's head is provided to the eyecups 21. The elastic band 230 of the buckle device 200 may be used as said head strap. A piece of the elastic band 230 passes through an outer end of each eyecup 21. As the both end portions of the elastic band 230 extending from the respective sides of the buckle holder 210 of the buckle device 200 is pulled and then released, the elastic band 230 can be locked to the buckle holder 210 while appropriately pressing the wearer's head.

Dissimilar to the depiction in FIG 15, two pieces of the elastic bands 230 may be used. In such a case, one end portion of each elastic band 230 passes through the buckle holder 210, while the opposite end thereof may be joined to the respective eyecups 21. Further, the buckle device 200 may be applied to different types of goggles other than the swimming goggle shown in FIG. 15.

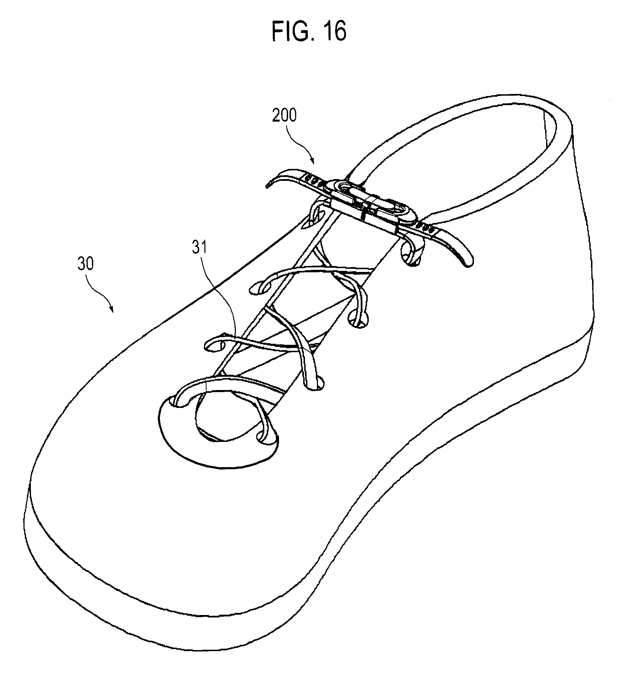

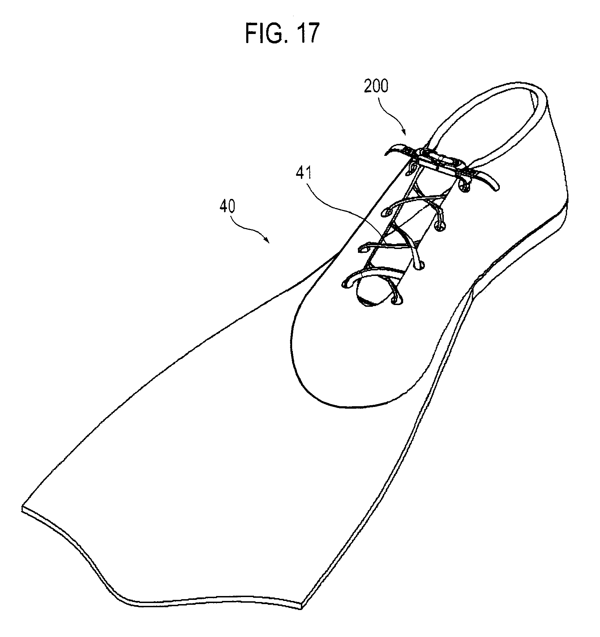

The buckle device 200 may be applied to footwear. As shown in FIG. 16, the buckle device 200 may be applied to a shoe 30 using a string 31 for fastening. Further, as shown in FIG. 17, the buckle device 200 may be applied to a swim fin 40 using a string 41 for fastening. In the application examples shown in FIGS. 16 and 17, the string 31, 41 may comprise the elastic band 230 in its entirety. Alternatively, a middle part of the string 31, 41 may comprise fabric, while only a portion of its both end portions may comprise the elastic band 230. By using the buckle device 200, the string 31 of the shoe 30 or the string 41 of the swim fin 40 can be easily adjusted.

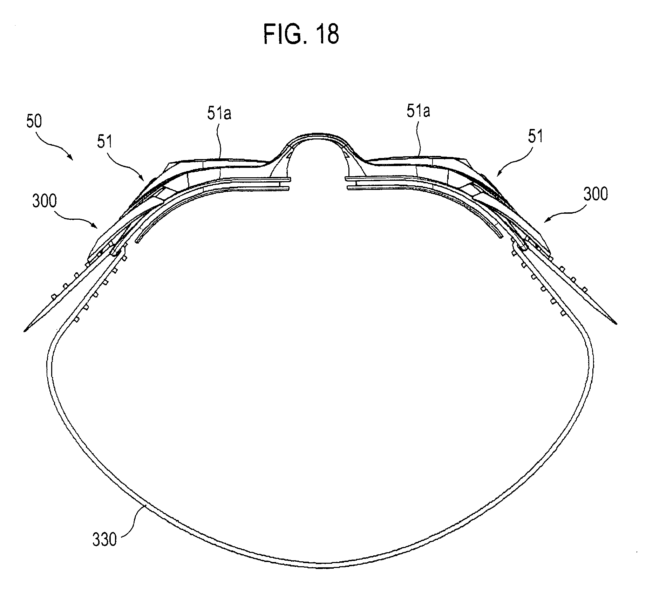

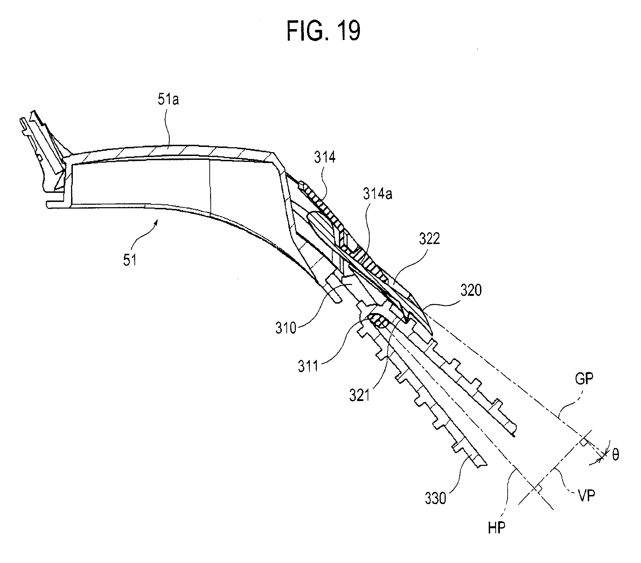

FIGS. 18 and 19 show a swimming goggle as an example of the article to which the buckle device according to the first embodiment is applied. The swimming goggle 50 includes a pair of eyecups 51 with a lens portion 51a respectively. A buckle device 300, which is configured similar to the buckle device according to the first embodiment, is attached to an outer end of each eyecup 51. This example employs a piece of an elastic band 330. Each end portion of the elastic band 330 extends through a buckle holder 310 as going around a locking pin 311 of the buckle device 300 attached to each eyecup 51. Referring to FIG. 19, the buckle holder 310 is disposed in the outer end of the eyecup 51. The buckle holder 310 may be integrally formed with the eyecup 51. Alternatively, the buckle holder may be made separately and be attached to the eyecup 51 through fitting. A guide portion 314 guiding a slide movement of a slide buckle 320 is formed on the buckle holder 310. The guide portion 314 has a flat guide surface 314a on its bottom surface (its surface facing toward the locking pin 311). The slide buckle 320 includes a recess 322 on which the guide portion 314 is placed. The buckle holder 310 is configured such that the guide surface 314a comes into surface contact with the surface of the recess 322 to guide the slide buckle 320. The locking pin 311 is integrally formed with the buckle holder 310 in the vicinity of the outer end of the buckle holder 310.

The guide surface 314a of the guide portion 314 is inclined with respect to the locking pin 311. Specifically, the guide surface 314a is situated in a guide plane GP slantingly intersecting a second plane VP, which is orthogonal to a first plane HP passing through a central point CP of the locking pin 311, at a predetermined angle θ with respect to the first plane HP. Accordingly, a ratchet protrusion 321 approaches and is moved away from the locking pin 311 obliquely with respect to the first plane HP while the slide buckle 320 is guided along the guide surface 314a. By pulling or releasing the elastic band 330, the slide buckle 320 is slidably moved between a locked position where the elastic band 330 is compressed by the ratchet protrusion 321 and is fixed to the locking pin and an unlocked position where the elastic band 330 is allowed to move between the ratchet protrusion 321 of the slide buckle 320 and the locking pin 311.

While the present invention has been described herein with reference to the foregoing embodiments and the accompanying drawings, the present invention should not be limited thereto. It will be apparent to those of ordinary skill in the art that various substitutions, alternations and modifications may be made without departing from the scope of the present invention.

## Claims

1. A buckle device, comprising:
a pair of elastic bands including a plurality of engaging portions in a lengthwise direction;
a buckle holder including: a pair of locking pins apart from each other, the pair of the elastic bands being hung on the locking pins respectively; and a pair of guide surfaces each inclined in opposite directions with respect to a plane passing through the pair of the locking pins; and
a pair of slide buckles each slidably fitted to the buckle holder, the slide buckle including: a ratchet protrusion configured to engage the engaging portion of the elastic band; and a slide surface configured to come into surface contact with the guide surface of the buckle holder,
wherein the slide buckle is movable between an unlocked position where the engaging portion pushes the ratchet protrusion in a first direction to allow a movement of the elastic band between the ratchet protrusion and the locking pin, and a locked position where the engaging portion pushes the ratchet protrusion in a second direction opposite to the first direction so that the ratchet protrusion presses the elastic band against the locking pin.

2. The buckle device of Claim 1, wherein the buckle holder includes a pair of wall portions,
wherein the locking pin and the guide surface are located between the wall portions, and
wherein the slide buckle is slidably fitted between the guide surface and the wall portions.

3. The buckle device of Claim 2, wherein the buckle holder includes a pair of guide tongues extending toward the locking pins respectively, a surface of the guide tongue facing toward the locking pin comprising the guide surface, and
wherein the slide buckle includes a recess on which the guide tongue is placed, a surface of the recess comprising the slide surface.

4. The buckle device of Claim 3, wherein an edge of the wall portion facing toward the guide surface is inclined at a same inclination as an inclination of the guide surface.

5. The buckle device of Claim 1, wherein the guide surface is inclined with respect to the plane such that the elastic band is compressed against the locking pin in the locked position of the slide buckle and does not move in the second direction.

6. The buckle device of Claim 1, wherein the buckle holder includes a push button movable perpendicularly to the plane between the locking pins, the push button including a pair of inclined surfaces each inclined as opposed to the guide surface,
wherein the slide buckle includes an inclined surface contacting the inclined surface of the push button in the locked position, and
wherein when the push button is moved to the guide surface the inclined surface of the push button pushes the inclined surface of the slide buckle in the first direction to move the slide buckle to the unlocked position.

7. A buckle device, comprising:
an elastic band including a plurality of engaging portions in a lengthwise direction;
a slide buckle including: a ratchet protrusion configured to engage the engaging portion of the elastic band; and a slide surface; and
a buckle holder slidably supporting the slide buckle, the buckle holder including: a locking pin on which the elastic band is hung; and a guide surface configured to come into surface contact with the slide surface of the slide buckle,
wherein the guide surface is situated in a guide plane intersecting a second plane obliquely with respect to a first plane passing through a center of the locking pin, the second plane being orthogonal to the first plane, and
wherein the slide buckle is movable between an unlocked position where the engaging portion pushes the ratchet protrusion in a first direction to allow a movement of the elastic band between the ratchet protrusion and the locking pin, and a locked position where the engaging portion pushes the ratchet protrusion in a second direction opposite to the first direction so that the ratchet protrusion presses the elastic band against the locking pin.

8. The buckle device of Claim 7, wherein the buckle holder includes a guide portion extending toward the locking pin, a surface of the guide portion facing toward the locking pin comprising the guide surface, and
wherein the slide buckle includes a recess on which the guide portion is placed, a surface of the recess comprising the slide surface.

9. The buckle device of Claim 1 or 7, wherein the engaging portion of the elastic band includes one selected from a protrusion, a ridge and a groove, the protrusion, the ridge and the groove extending in a width direction of the elastic band.

10. A swimming goggle comprising a pair of eyecups with a lens portion and the buckle device according to Claim 1, wherein the elastic band of the buckle device is joined to an outer end of the eyecup.

11. A shoe comprising a string for fastening and the buckle device according to Claim 1, wherein the elastic bands of the buckle device are coupled to both ends of the string.

12. A swim fin comprising a string for fastening and the buckle device according to Claim 1, wherein the elastic bands of the buckle device are coupled to both ends of the string.

13. A swimming goggle, comprising:
a pair of eyecups with a lens portion respectively;
the buckle device according to Claim 7 attached to an outer end of one of the pair of the eyecups; and
the buckle device according to Claim 7 attached to an outer end of the other of the pair of the eyecups,
wherein the elastic bands of the buckle devices are integrally formed.
